# FASCICULE DE BREVET EUROPEEN

(11) **EP 1 526 850 B1**
(45) Date de publication et mention de la délivrance du brevet: **27.08.2008**
(21) Numéro de dépôt: 03753643.0
(22) Date de dépôt: 15.07.2003
(51) Int. Cl.: A61K 31/375, A61P 25/02

(54) **Utilisation de la vitamine C pour le traitement de la maladie de Charcot-Marie-Tooth**
Verwendung von Vitamin C zur Behandlung der Charcot-Marie-Tooth Krankheit
Use of vitamin C for the treatment of Charcot-Marie-Tooth disease

(30) Priorité: 16.07.2002 FR 0208966
(43) Date de publication de la demande: 04.05.2005
(73) Titulaire: Université de la Méditerranée, Aix-Marseille II, 13284 Marseille Cedex 07 (FR); INSTITUT NATIONAL DE LA SANTE ET DE LA RECHERCHE MEDICALE (INSERM), 75654 Paris Cédex 13 (FR); Association Française contre les Myopathies, 75651 Paris Cedex 13 (FR)
(72) Inventeur: FONTES, Michel, F-13710 Fuveau (FR); PASSAGE, Edith Résidence Ste Germaine, F-13012 Marseille (FR); SANGUEDOLCE, Véronique Résidence Valmante, F-13009 Marseille (FR); NOREEL, Jean-Chrétien, F-13008 Marseille (FR)
(74) Mandataire: Tezier Herman, Béatrice
(86) Numéro de dépôt international: PCT/FR2003/002236
(87) Numéro de publication internationale: WO 2004/006911

(56) Documents cités:
- EP-A- 0 694 302
- EP-A- 0 797 993
- EP-A- 0 820 770
- WO-A-95/01096
- GB-A- 890 638
- US-A- 6 114 388
- DATABASE CA [en ligne] CHEMICAL ABSTRACTS SERVICE, COLUMBUS, OHIO, US; MATSUSHITA, KAZUO ET AL: "Experimentally-induced toxic effects in the retina upon the regeneration of visual purple" retrieved from STN Database accession no. 62:46556 HCA XP002236327 & NIPPON GANKA GAKKEI ZASSHI ( 1962 ), 66(9), 687-94,
- DATABASE CA [en ligne] CHEMICAL ABSTRACTS SERVICE, COLUMBUS, OHIO, US; GIUBILEO, MASSIMO: "A case of Guillain - Barre syndrome in a worker exposed to lead" retrieved from STN Database accession no. 49:80961 HCA XP002236328 & MED. LAVORO ( 1955 ), 46, 162-6,
- VIKTORA P ET AL: "Treatment of Landry's ascending type of polyradiculoneuritis in a resuscitation department" BRATISLAVSKE LEKARSKE LISTY 1973, vol. 60, no. 6, 1973, pages 731-733, XP009008486
- WANG HONG ET AL: "Experimental and clinical studies on the reduction of erythrocyte sorbitol-glucose ratios by ascorbic acid in diabetes mellitus." DIABETES RESEARCH AND CLINICAL PRACTICE, vol. 28, no. 1, 1995, pages 1-8, XP002236325 ISSN: 0168-8227
- GROBER U: "Orthomolecular medicine: Usefulness of micronutrients in diabetes mellitus" DEUTSCHE APOTHEKER ZEITUNG 14 FEB 2002 GERMANY, vol. 142, no. 7, 14 février 2002 (2002-02-14), pages 46-52, XP002236326 ISSN: 0011-9857

## Description

### Introduction et état de l'art

La présente invention se rapporte aux domaines techniques de la biologie, de la pharmacologie et de la médecine. Ses applications concernent notamment les domaines de la santé humaine et animale. Plus particulièrement, l'invention décrit l'utilisation de la vitamine C dans la préparation de compositions destinées à la prévention ou au traitement de la maladie de Charcot-Marie-Tooth

Sous le terme de maladies neuromusculaires, on regroupe toutes les affections qui atteignent les cellules et les fibres du système nerveux périphérique (atteinte neurogène), qui atteignent les muscles (atteinte myogène), ou enfin qui touchent la jonction entre les fibres nerveuses et les fibres musculaires (maladie de la transmission neuromusculaire). Au sein des maladies neuromusculaires, on réserve le terme de neuropathies aux atteintes des fibres nerveuses aboutissant à la destruction des neurones.

Dans les atteintes neurogènes, on peut distinguer trois sous-groupes selon le type fonctionnel de cellules ou fibres nerveuses atteintes :
- Les atteintes motrices pures, par exemple les amyotrophies spinales. Elles sont liées à des anomalies des neurones moteurs du système nerveux périphérique (SNP). Il existe aussi d'autres maladies neurogènes purement motrices, dans lesquelles les motoneurones sont indemnes, mais où ce sont les fibres motrices des nerfs qui sont spécifiquement atteintes.
- Les atteintes sensitives pures concernent les fibres sensitives des nerfs du système nerveux périphérique.
- Les neuropathies sensori-motrices ou sensitivo-motrices atteignent les nerfs moteurs et sensitifs. Ces neuropathies sont de loin les plus fréquentes.

Les neuropathies sensitivo-motrices peuvent être d'origine non héréditaire ou au contraire d'origine héréditaire comme par exemple la maladie de Charcot-Marie-Tooth (CMT).

La maladie de CMT découverte en 1886 est la plus fréquente des neuropathies sensitivo-motrices héréditaires. On distingue plusieurs maladies de CMT : les types 1 et 2 sont le plus souvent autosomiques dominants mais peuvent également être hérités selon un mode autosomique récessif. Plus rarement, le type 1 peut se transmettre de façon dominante liée au chromosome X.
La maladie de CMT présente ainsi une grande hétérogénéité génétique, mais il a été démontré, en 1991, que la majorité des malades atteints de la forme CMT1A la plus fréquente (plus de la moitié des cas) présentaient une duplication de la région 17p11.2 [Lupski, 1991 #1] [Raeymaekers, 1991 #2], de taille invariante (1,5 Mb), qui comprend le gène codant pour la protéine de la myéline PMP22. Des mutations ponctuelles ayant été retrouvées chez de rares patients CMT1A ne présentant pas cette duplication ont permis de démontrer que ce gène est la cause principale du phénotype CMT1A.

Le moment où apparaissent les premiers symptômes est très variable d'un patient à l'autre et pour chaque type de maladie de CMT. De même, la sévérité avec laquelle le gène responsable de la maladie s'exprime dépend de plusieurs facteurs. Ainsi, au sein d'une même famille, la sévérité de la maladie peut différer d'un individu à l'autre.
Plusieurs composants différents du système nerveux périphérique peuvent être atteints. Une classification de ces maladies a été proposée à partir des éléments atteints :

Les CMT de type 1 se caractérisent par des anomalies des gaines de myéline. Ces dernières sont souvent très fines et parfois totalement absentes. Le processus qui conduit à la perte de myéline s'appelle la démyélinisation. Quand la gaine de myéline est atteinte, la conduction saltatoire de l'influx nerveux ne peut plus avoir lieu de façon correcte et la vitesse de conduction nerveuse diminue. Parfois, l'influx nerveux n'arrive plus du tout. Ceci se produit en particulier pour les axones les plus longs à savoir ceux qui atteignent les extrémités du corps (parties distales) et innervent les pieds et les mains. C'est pour cette raison que ces maladies de CMT se manifestent préférentiellement dans les membres et en particulier à leurs extrémités.
Lorsqu'il y a démyélinisation, des processus de réparation de la gaine de myéline peuvent prendre place (remyélinisation). Ces phénomènes alternant de dé- et de remyélinisation peuvent causer la formation progressive de structures concentriques dont l'aspect évocateur au microscope les fait qualifier de bulbes d'oignon. Dans les bulbes d'oignon, le nombre de cellules de Schwann entourant les axones est fortement augmenté, les fibres nerveuses deviennent donc hypertrophiques.

Pour les CMT de type 2, l'anomalie se trouve dans les axones eux-mêmes. Le nombre d'axones diminuant (dégénérescence axonale), le nombre de fibres nerveuses diminue. Parfois, il y a une régénération des axones atteints. Ce phénomène est appelé bourgeonnement axonal.

Dans les CMT de type 1, les vitesses de conduction nerveuse sont fortement ralenties. Dans les CMT de type 2, les anomalies des vitesses de conduction nerveuse sont mineures ou absentes.

L'âge d'apparition des symptômes pour les CMT de type 1 et 2 peut varier chez l'homme de la première à la sixième décennie de vie. La CMT de type 2 se révélerait en moyenne plus tard que le type 1, rarement avant l'âge de 10 ans. Cependant, il est habituellement impossible de distinguer ces deux types de CMT sur les seuls symptômes cliniques.

Quel que soit l'âge, les anomalies vont d'abord concerner les parties du corps les plus distales à savoir les pieds (pieds creux) et les orteils. Si la maladie évolue, l'atteinte touchera les muscles de la partie antérieure et externe de la jambe, provoquant leur atrophie (on parle notamment de « mollets de coq »), et parfois les muscles des mains. Dans ce dernier cas, les mouvements fins des doigts et la réalisation de gestes nécessitant à la fois force et précision, peuvent devenir difficiles. Dans certains cas, les muscles des cuisses, des épaules et du dos pourront être touchés ultérieurement. Les patients peuvent assez souvent présenter un tremblement lié à l'excès de travail musculaire demandé à des muscles affaiblis par la maladie. Dans tous les cas, la maladie évolue lentement, de façon progressive sur des années, voire pas du tout. Quelle que soit la partie du corps qui est touchée, cette atteinte est toujours symétrique.

Comme pour la plupart des affections neuromusculaires, en particulier les affections héréditaires, la guérison de la CMT n'est actuellement pas possible et aucune thérapeutique curative n'a jusqu'ici été proposée, les patients ne bénéficiant que de traitements symptomatiques pour diminuer le plus possible les conséquences désagréables de la maladie. Parmi les médicaments symptomatiques, les dérivés de la quinine sont utilisés contre les crampes musculaires. Lorsque les antalgiques usuels (Aspirine, Paracétamol, etc.) sont inefficaces, le traitement des douleurs est parfois amélioré par des médicaments tels que certains anti-dépresseurs ou anti-épileptiques. La chirurgie est également parfois envisagée pour corriger les déformations ostéo-articulaires sources de douleurs et d'inconfort.

A côté des CMT 1 et 2, on distingue d'autres maladies héréditaires qui comportent des symptômes communs à ceux décrits pour la maladie de CMT, associés à d'autres anomalies. II s'agit notamment des neuropathies héréditaires avec hypersensibilité à la pression nerveuse, de la maladie de Refsum, de la maladie de Strümpell-Lorrain, de la rétinite pigmentaire, etc.

La présente invention propose pour la première fois des compositions permettant de prévenir et de traiter la maladie de Charcot-Marie-Tooth. Ceci est permis de façon surprenante par l'administration de compositions comprenant de la vitamine C.

Les bienfaits de l'acide ascorbique ou vitamine C sont nombreux et son utilisation est largement recommandée dans le cadre de divers traitements thérapeutiques. Une partie des actions biologiques connues de la vitamine C est expliquée par son action anti-oxydante, qui aide à lutter contre les méfaits des radicaux libres. Elle participe aussi à la synthèse des neurotransmetteurs sécrétés par le cerveau et par les terminaisons nerveuses. Parmi ces derniers, on peut citer notamment les catécholamines qui interviennent dans les réactions de stress. La vitamine C est également nécessaire au développement et à la bonne santé des muscles et des os. Elle accélère les processus de cicatrisation, aide au fonctionnement du système immunitaire (d'où sa large utilisation anti-infectieuse), combat les réactions allergiques, intervient dans la synthèse d'hormones et dans l'absorption du fer. Elle joue un rôle dans la détoxification de l'organisme en stimulant la fabrication d'une enzyme, le cytochrome P450. Elle s'oppose en outre à l'action de composés cancérigènes que sont les nitrosamines. La vitamine C est par exemple utilisée pour favoriser la reconstitution du collagène, pour lutter contre les maladies bactériennes ou virales ou pour lutter contre les problèmes liés à l'athérosclérose, à l'hypertension, aux hémorroïdes ou au diabète. Elle est également utilisée comme régulateur de la digestion.

L'invention décrit à présent une nouvelle utilisation particulièrement avantageuse de la vitamine C dans le cadre de la préparation de compositions pharmaceutiques destinées à la prévention ou au traitement de la maledie de Charcot-Marie - Tooth.

### Description générale de l'invention

Le problème que la présente invention se propose de résoudre consiste à offrir aux patients atteints ou aux sujets présentant des risques de développer la maladie de Charcot-Marie-Tooth des compositions destinées à la prévention ou au traitement de ladite maladie.

### Description détaillée de l'invention

La présente invention concerne ainsi l'utilisation de la vitamine C ou d'un dérivé de cette dernière dans le cadre de la préparation d'une composition destinée à la prévention ou au traitement de la maladie de Charcot-Marie-Tooth.

Sans vouloir se lier à une quelconque théorie concernant le mécanisme d'action de la vitamine C, les observations réalisées par les inventeurs dans le cadre de leurs travaux leur ont permis d'envisager différentes hypothèses. Les inventeurs ont récemment démontré que l'expression du gène PMP22 codant pour une protéine de la gaine de myéline se trouve sous le contrôle direct de l'AMPc, par l'intermédiaire de la fixation de CREB sur deux sites du promoteur du gène, situés à 1,5 kb du site d'initiation de la transcription. En l'absence d'AMPc, l'activité du promoteur minimal schwannien spécifique (300 pb) est inhibée. Le traitement à l'aide d'AMPc permet de lever cette inhibition et de restituer l'expression du promoteur minimal. L'une des hypothèses envisagées par les inventeurs était qu'une utilisation d'une quantité au contraire réduite d'AMPc pouvait diminuer l'activité de ce promoteur et ainsi diminuer la sur-expression de PMP22. Les inventeurs ont par ailleurs constaté que la sévérité du phénotype semblait dépendre du niveau de sur-expression, avec un effet de seuil. En effet, une sur-expression de 70 % ne semble pas pathogène alors qu'une sur-expression de 100% (patients CMT1A) semble l'être. Dans le cadre de la présente invention, les inventeurs ont testé l'action de la vitamine C sur le pool endogène d'AMPc et ont constaté que la diminution de ce pool permettait de diminuer l'expression de PMP22, de manière à ce que son taux passe au dessous du seuil de pathogénéicité. Cette hypothèse a pu être validée chez des animaux traités pendant 3 mois avec un inhibiteur de l'AMPc à savoir la vitamine C et chez des animaux de la même fratrie auxquels avait été administré un placebo. Ces animaux ont été sacrifiés. Les nerfs sciatiques ont été prélevés, l'ARN extrait et le niveau d'expression testé en PCR temps réel, à l'aide d'amorces spécifiques du transcrit humain. Les résultats montrent que le niveau d'expression de PMP22 est diminué après le traitement par la vitamine C. Le nerf sciatique des souris traitées contient ainsi 8 fois moins d'ARN messager de PMP22 que celui des souris placebo (cf. : matériels et méthodes).

L'invention concerne ainsi l'utilisation de la vitamine C ou d'un dérivé de cette dernière pour la préparation d'une composition capable de réguler l'expression de l'AMPc et/ou l'expression de la protéine PMP22 constitutive de la gaine de myéline entourant les fibres nerveuses. Selon un mode préféré de réalisation de l'invention, la composition selon l'invention diminue l'expression de l'AMPc et/ou celle de la protéine PMP22 laquelle est généralement sur-exprimée chez les sujets atteints de neuropathies périphériques, en particulier chez les sujets atteints de la maladie CMT1.

L'invention concerne par ailleurs une méthode de préparation d'une composition destinée au traitement de la maladie de Charcot-Marie-Tooth **caractérisée en ce que** la composition comprend, à titre de substance active de l'acide ascorbique ou un dérivé de ce dernier assimilable par l'homme ou l'animal, en association avec un véhicule acceptable sur le plan pharmaceutique.

Dans le cadre des utilisations et méthodes décrites ci-dessus, la vitamine C est choisie dans le groupe comprenant la vitamine C naturelle, la vitamine C synthétique et un mélange de ces dernières. La vitamine C naturelle peut être extraite d'un produit naturel et notamment de produits tels que l'Acérola, la baie d'églantier, la goyave, le persil, le cassis, le kiwi, le fenouil, la papaye, le choux-fleur cru, le brocoli cuit, l'orange, le cresson, le choux-rouge, la pomme de terre, la mangue, les jus de citron et de pamplemousse, la groseille, la framboise, le fruit de la passion, la myrtille, etc. ou encore de produits enrichis artificiellement en vitamine C. Il est également possible d'utiliser un dérivé de la vitamine C tel que des esters de vitamine C ou des sels de vitamine C. En particulier, les esters de la vitamine C peuvent être des esters d'ose de l'acide ascorbique, tels que notamment les dérivés glycosylés, mannosylé, fructosylé, fucosylé, galactosylé, N-acétylglucosaminé, N-acétylmuramique de l'acide ascorbique et leurs mélanges et plus spécialement l'ascorbyl-2 glucoside ou 2-O-alpha-D glucopyranosyl de l'acide ascorbique ou encore le 6-O-beta-D galactopyranosyl de l'acide L-ascorbique. Ces derniers composés ainsi que leurs procédés de préparation sont en particulier décrits dans les documents EP-A-487404, EP-A-425066 et J05213736. Les esters de la vitamine C peuvent être des esters tels que l'ascorbyl palmitate ou le dipalmitate L-ascorbate. D'autres dérivés de la vitamine C utilisables dans le cadre de la présente invention peuvent être des sels métalliques d'acide ascorbique phosphorylés, tels que notamment les ascorbyl phosphates de métal alcalin, les ascorbyl phosphates de métal alcalino-terreux et les ascorbyl phosphates de métal de transition. On utilise avantageusement l'ascorbyl phosphate de magnésium. Il peut également s'agir d'ascorbyl sulfates.

L'invention concerne également une méthode telle que décrite ci-dessus caractérisée en ce que la composition est un complément alimentaire adapté à la consommation animale, de préférence à la consommation humaine.

Un avantage particulièrement intéressant de l'utilisation selon l'invention est qu'elle peut faire intervenir comme principe actif une molécule, la vitamine C, peu coûteuse, facile à obtenir et utilisable immédiatement dans des essais cliniques de phase III.

Les compositions utilisées dans les méthodes selon l'invention comprennent en effet à titre de principe actif, en tant qu' inhibiteur de l'AMPc, de l'acide ascorbique ou un dérivé de ce dernier en association avec un véhicule acceptable sur le plan pharmaceutique. Elles sont destinées à prévenir ou à traiter la maladie de Charcot-Marie-Tooth. Une telle composition peut être une composition pharmaceutique ou un complément alimentaire adapté à la consommation animale, de préférence à la consommation humaine. La composition selon l'invention comprend généralement entre 250 mg et 6 grammes d'acide ascorbique ou d'un dérivé de ce dernier, de préférence entre 500mg (ou 1g) et 6 grammes, encore plus préférentiellement entre 3 et 5 grammes.

La quantité de princle actif à administrer dans le traitement de la maladie de Charcot-Marie-Tooth selon l'invention dépend bien évidemment de la nature et de la gravité de l'affection à traiter ainsi que du poids du malade. Néanmoins, les doses unitaires préférées comprendront généralement de 250 mg à 6 grammes de vitamine C, avantageusement de 1 à 6 grammes, encore plus préférentiellement de 3 à 5 grammes. Ces quantités n'excluent pas l'absorption de quantités plus élevées de vitamine C ou de dérivés de la vitamine C. La vitamine C est en effet une molécule bien connue dont les effets secondaires, peu nombreux, sont également connus (la lithiase en particulier). La vitamine C est classiquement considérée comme favorisant la formation de calculs rénaux (type oxalate) du fait de sa transformation en oxalate au cours de son catabolisme et du fait de l'acidification des urines. Des études de cohorte (« The health effect of vitamin C supplementation » : a review. Bendich et al., J Am Coll Nutr, 1995, 14, 124-136 ; "No contribution of ascorbic acid to renal calcium oxalate stones." Gester H, Ann Nutr Metab, 1997, 41, 269-82; « Biomarkers for establishing a tolerable upper intake level of vitamin C. » Johnston CS. Nutr Rev, 1999, 57, 71-7 ; CURHAN GC, WILLETT WC, RIMMEB et al, "A prospective study of the intake of vitamins C and B6 and the risk of kidney stones in men." The Journal of Urology, 1996, vol 155: 1847-1851 ; CURHAN GC, WILLET WC, SPEIZER FE et al, "Intake of vitamins B6 and C and the risk of kidney stones in women." J.AM.Soc.Nephrol., 1999, 10: 840-845; GERSTER H, "No contribution of ascorbic acid to renal calcium oxalate stones. Ann.Nutr.Metab.", 1997, 41: 269-282) ont cependant montré que la prise quotidienne de doses allant jusqu'à 1500 mg de vitamine C n'augmente pas le risque de calcul rénal. Certaines publications font état d'administrations de doses très élevées par voie orale de l'ordre de 20 à 40 grammes sans qu'il n'ait pu être constaté d'effet particulier. La pharmacocinétique de l'acide ascorbique explique que la prise de fortes doses de ce principe actif n'augmente ni la toxicité, ni le risque de formation de calculs rénaux. En effet, l'absorption gastro-intestinale se fait par un transporteur Na+ dépendant et est saturable. Pour 1 gramme de vitamine C, 75% est absorbé. Pour 5 grammes, 20% est absorbé. De plus, la transformation métabolique de l'acide ascorbique en oxalate est un phénomène limité : 1% de l'acide ascorbique urinaire est métabolisé en oxalate (Campbell G.D., Steinberg M.H., Bower J.D., « Ascorbic acid-induced hemolysis in G-6-PD deficiency », Ann. Of inter. Med, 1975, 82(6), 810 ; Rees D.C., Kelsey H., Richards J.D.M., « Acute haemolysis induced by high dose ascorbic acid in glucoe-6-phosphate dehydrogenase deficiency », Br. J. Med. ; 1993, 306, 841-2). Ainsi, des quantités bien plus importantes que celles décrites précédemment peuvent être absorbées sans risque par l'homme ou l'animal.
Les doses unitaires seront normalement administrées une fois par semaine, mais on peut également envisager des administrations plus rapprochées dans le temps, par exemple tous les 2 à 3 jours ou 1 à 5 fois par jour. Dans ce cas, les doses unitaires comprennent typiquement 0,1 à 1 gramme de vitamine C. Les doses administrées peuvent correspondre, selon un mode préféré, à une administration d'environ 250 mg (ou environ 500mg) par jour pendant 6 jours suivie d'une administration de 5 grammes (deux comprimés de 2,5 grammes par exemple) (ou environ 3 ou 4g) le septième jour. L'utilisation de vitamine C dans le cadre du traitement de la maladie de Charcot-Marie-Tooth est de préférence prolongée dans le temps. Cette administration se traduit généralement par des cures prolongées dans le temps qui peuvent être répétées durant la vie du patient. Il peut s'agir de cures de plusieurs mois, de préférence de deux mois à six mois, voire 18 mois. Dans les cas les plus sévères, cette prise de vitamine C peut se poursuivre pendant plusieurs années voire toute la vie du patient.

L'acide ascorbique ou ses dérivés peuvent être absorbés en dehors des repas (avant ou après) ou pendant ces derniers. L'administration d'une composition selon l'invention à base de vitamine C s'effectue préférablement par voie orale. Elle peut néanmoins également s'effectuer par voie entérale ou parentérale dans un véhicule approprié. La vitamine C se présente sous une forme (solide ou liquide) adaptée à une telle administration. La vitamine C est en effet une molécule sensible à la lumière et facilement oxydable en particulier en milieu aqueux. En revanche, dans un milieu anhydre, sa solubilité est considérablement réduite.
La vitamine C utilisée dans le cadre de la présente invention se présente avantageusement sous une forme solide. Lorsqu'il s'agit des sels de l'acide ascorbique, ces derniers sont administrés de préférence sous une forme soluble dans l'eau ou dans toute autre boisson. Des alcools compatibles avec l'eau tels que le propylène glycol, le polypropylène glycol et le glycérol ont été utilisés comme véhicules capables d'augmenter la stabilité de la vitamine C dans l'eau [cf. brevet US n° 4,983,382 (Wilmott et Znaiden)] et peuvent également être utilisés dans le cadre de la présente invention.

La vitamine C peut ainsi se présenter sous forme de solutions aqueuses contenant les doses unitaires indiquées précédemment, dans un véhicule de type alcool ou encore dans un véhicule isotonique ou stérile qui contient éventuellement des agents de dispersion et/ou des mouillants compatibles sur le plan pharmacologique. Il est également envisageable d'associer l'administration de la vitamine C avec d'autres composés capables de potentialiser son action, tels que des dérivés ou des solutions tamponnées. Ces compositions sont conditionnées de manière appropriée (gélule, solution injectable, comprimé, etc.) pour permettre une administration orale, entérale ou encore une administration parentérale, par exemple une administration intraveineuse, intra-musculaire ou sous-cutanée, l'introduction dans un dispositif de perfusion intraveineuse ou à la surface d'une membrane de dialyse, ou bien encore une administration par un système d'implant permettant la perfusion sous-cutanée.
On peut également administrer les composés selon l'invention par voie rectale ou bien percutanée. Dans ce cas, les formes unitaires d'administration sont préparées de manière conventionnelle selon les techniques classiques connues de l'homme du métier, avec les excipients couramment utilisés dans ce domaine.

Quelle que soit la voie d'administration choisie, des compositions à base de vitamine C préférée selon l'invention se présentent sous une forme favorable à la protection et à l'assimilation optimale du principe actif.

L'invention concerne par ailleurs des outils et kit destinés à la mise en oeuvre de l'une ou l'autre des méthodes telles que décrites ci-dessus.

Les propriétés avantageuses de la vitamine C dans le traitement de la maladie de Charcot-Marie-Tooth sont illustrées dans la section suivante par des données pharmacologiques et des exemples qui doivent être considérés comme illustratifs et non limitatifs.

### LEGENDES DES FIGURES

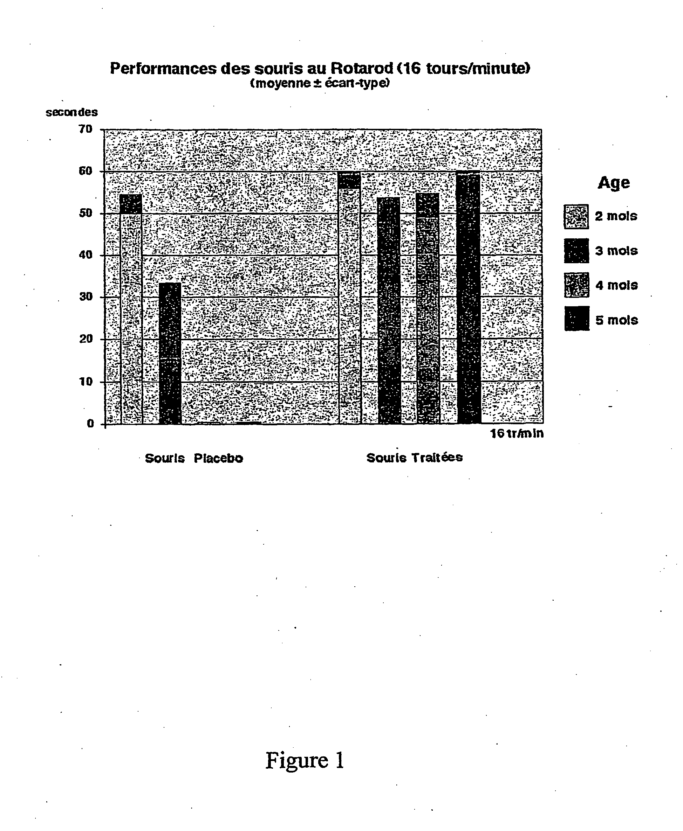

Figure 1 : Des mâles âgés de 2 mois et provenant de deux fratries ont été traités soit avec un placebo (4 animaux) soit avec de la vitamine C (6 animaux) pendant trois mois. Leurs performances au test du rotarod ont été estimées tous les mois (à 3 mois, 4 mois et 5 mois). L'écart type est représenté par la partie supérieure grisée de chaque colonne.

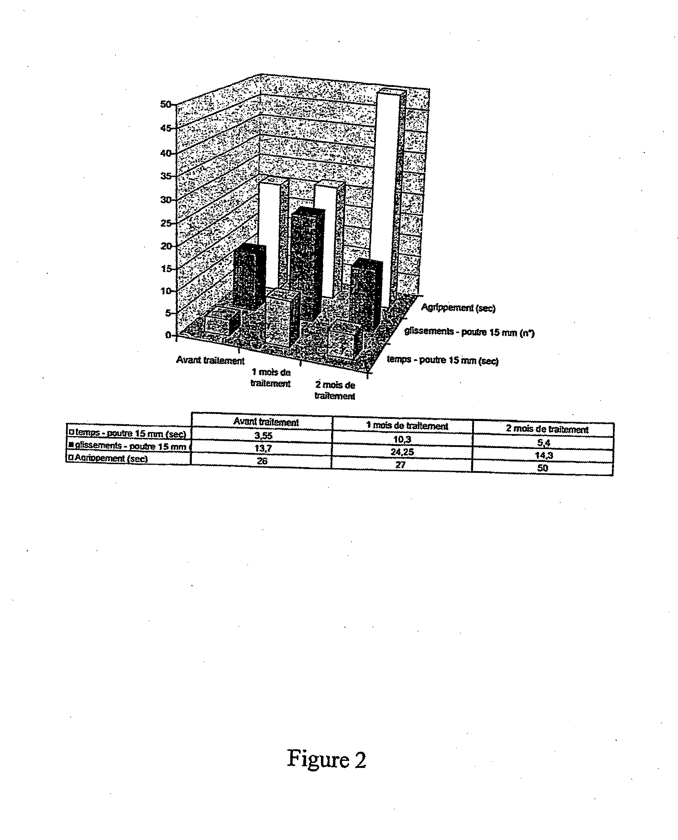

Figure 2: Une seconde série d'essais pré-cliniques désignés de manière identique à ceux de la première série (décrite dans la figure 1), a été réalisée. Les animaux ont été testés après un mois et deux mois de traitement en utilisant le test de la poutre (plus le temps mis par l'animal à parcourir le trajet est long ou bien plus il effectue de glissements, moins ses capacités locomotrices sont performantes). Les animaux ont également été testés en utilisant le « grip test » qui mesure la force de traction nécessaire à appliquer afin que l'animal lâche la barre (plus elle est élevée plus l'animal est performant).

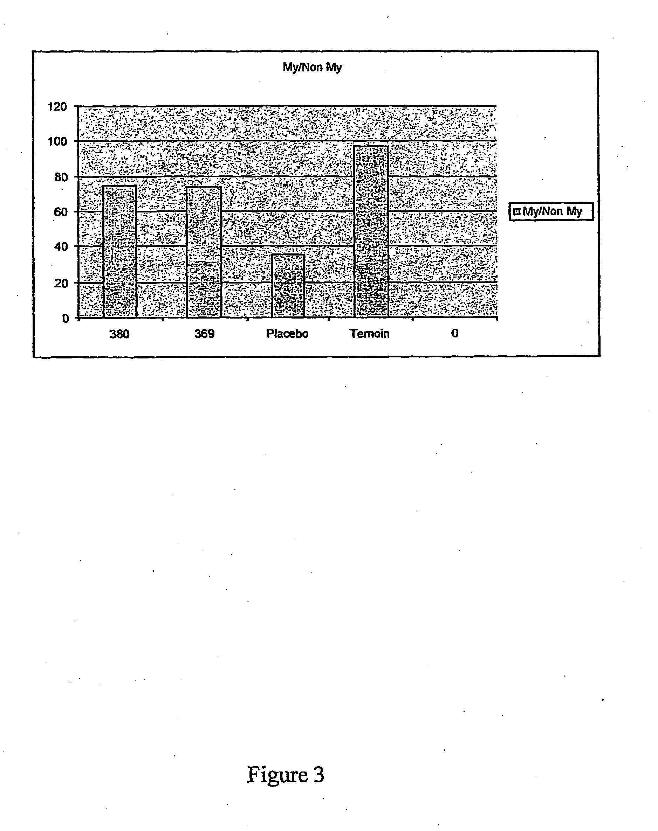

Figure 3 : Des animaux traités par la vitamine C pendant trois mois (369 et 380) ou traités par un placebo ont été sacrifiés. Le nerf sciatique a été prélevé et le pourcentage de fibres myélinisées a été estimé par examen histologique et apparaît sur la figure en ordonnée de même que les résultats d'un témoin non transgénique (plus de 95% des fibres nerveuses périphériques sont myélinisées chez l'adulte).

### MATERIELS ET METHODES

La maladie de Charcot-Marie-Tooth (CMT) est la forme la plus fréquente des neuropathies périphériques héréditaires, affectant un individu sur 2500.

Afin de mieux comprendre la physiopathologie de cette maladie et de proposer des solutions thérapeutiques, un modèle murin de cette maladie a été construit en 1996 par transgènose d'un YAC humain contenant le gène PMP22 (Huxley et al., Human Molecular Genetics, 1996, Vol. 5, No. 5, 563-569). L'exploration de ce modèle a montré sa pertinence par rapport à la maladie humaine (Huxley et al., Human Molecular Genetics, 1998, Vol. 7, No. 3, 449-458). L'action de la vitamine C a ainsi pu être testée sur ce dernier.

Un premier essai « ouvert » a été effectué, sans critère d'inclusion, en séparant simplement mâles et femelles afin de pouvoir déceler rapidement si un effet de correction phénotypique était visible. Le critère retenu comme test de la force musculaire était le test du rotarod, classiquement utilisé en pharmacologie. 16 femelles et 12 mâles transgéniques C22 ont ainsi été testés comme témoins sans traitement (résultats présentés dans le tableau I).

**Tableau I :**

| | Animaux non traités | | Animaux traités | |
|---|---|---|---|---|
| | Nombre d'animaux | Secondes Sur la barre | Nombre d'animaux | Secondes Sur la barre |
| Mâles | 12 | 11.3 +/- 13 | 5 | 46 +/- 14 |
| Femelles | 16 | 25 +/- 8 | 12 | 45.7 +/-10 |
| Non Transgèniques | 20 | 60 | | |

12 femelles et 5 mâles C22 ont été traité avec la Vitamine C. Les résultats, exprimés en secondes, confirment que les mâles sont plus sévèrement atteints (il existe même une mortalité précoce des mâles dans cette lignée, puisque tous les mâles meurent avant l'âge de 10 mois). D'autre part, il apparaît clairement qu'aussi bien les mâles que les femelles traités ont des performances au rotarod bien supérieures à celles des animaux non traités. La correction semble partielle, au moins dans cet essai, car les mâles, aussi bien que les femelles, se retrouvent à un niveau de performance semblable et légèrement inférieur à celui des témoins.

Ces premiers résultats très encourageants ont conduit à effectuer une deuxième série d'expériences, effectuées comme un essai clinique humain, à savoir en double aveugle (Vitamine C contre placebo).
Seuls les mâles (issus de deux fratries), puisque le degré de sévérité est sexe-spécifique, ont été inclus, la moitié étant traitée avec un placebo, l'autre moitié avec la vitamine C à raison d'1.12 mg administré par semaine en une seule fois. Le fait de n'inclure que les mâles est très important car on sait qu'il existe, chez les patients, une grande variabilité de la sévérité de l'atteinte d'un sexe à l'autre, variabilité qui se retrouve également chez les modèles animaux utilisés, chez lesquels semble exister un gène modificateur majeur ayant un impact sur la myélinisation. Le «fond génétique» semble ainsi jouer un grand rôle sur la sévérité du phénotype. II était donc légitime de travailler sur des individus issus d'une même fratrie.

Les animaux ont été traités à partir de l'âge de deux mois (le phénotype pathologie locomotrice apparaissant vers l'âge d'un mois), à raison d'un gavage correspondant à l'administration d'1,12 mg par semaine, soit avec la Vitamine C soit avec le placebo. Les animaux ont ensuite été étudiés à l'aide du test au Rotarod. L'essai a duré trois mois (soit de l'âge de deux mois à l'âge de cinq mois pour les animaux). Les résultats sont présentés dans la Figure 1.

Ces résultats montrent que le premier mois de traitement n'est pas très efficace, même si la décroissance des performances au rotarod est moins importante pour les animaux traités que pour les témoins. Une amélioration spectaculaire est en revanche constatée dés le deuxième mois de traitement ; les animaux traités restant prés de 50 secondes sur la barre, alors que les animaux non traités n'arrivent pas à tenir du tout (des fratries au phénotype sévère avaient été sélectionnées).Les performances des animaux s'améliorent encore davantage au troisième mois.

Devant ces résultats spectaculaires, le traitement des animaux avec de la vitamine C à été maintenu et leurs performances ont été suivies. Ces dernières ont continué à s'améliorer, même en tenant compte du vieillissement des animaux. De plus, les animaux non sacrifiés (voir ci-dessous), âgés de 24 mois, ne sont pas morts, montrant ainsi une correction du phénotype létalité mâle.

Afin de confirmer ces résultats, et d'affiner le mécanisme de la correction phénotypique, un troisième essai a été réalisé. Deux tests ont été mis en oeuvre : un test de posture (traversée d'une poutre de plus en plus fine) et un test de force musculaire, le "grip test". Les résultats sur deux mois de traitement sont présentés dans la Figure 2. Il apparaît clairement que les performances à la traversée de la poutre se dégradent au premier mois, mais s'améliorent au deuxième, confirmant tout à fait les tests au rotarod. Quant au "grip test", il montre également une amélioration à partir du deuxième mois de traitement. Ce test représente une bonne mesure de la force musculaire. Les résultats confirment donc que le traitement à la vitamine C permet une récupération de la force musculaire, probablement par réacquisition de muscles fonctionnels.

Afin d'apporter des données sur le mécanisme de cette correction phénotypique, l'aspect anatomo-pathologique des nerfs des animaux traités a été examiné. Pour cela, un certain nombre d'animaux traités ont été sacrifiés, et une analyse histologique effectuée. Le pourcentage de fibres nerveuses myélinisées augmente avec le traitement (Figure 3). II est de 95% pour les animaux non transgéniques, de 20/30% pour les souris C22 non traitées et de 70% pour les animaux traités. Le traitement réactive donc la myélinisation, inhibée par la sur-expression de PMP22.

### Mécanisme moléculaire de la correction :

L'expression du gène PMP22 se trouve sous le contrôle direct de l'AMPc, par l'intermédiaire de la fixation de CREB sur deux sites du promoteur du gène, situés à 1;5 kb du site d'initiation de la transcription. En l'absence d'AMPc, l'activité du promoteur minimal schwannien spécifique (300 pb) est inhibée. Le traitement à l'aide d'AMPc permet de lever cette inhibition et de restituer l'expression du promoteur minimal. L'une des hypothèses envisagées était qu'une utilisation d'une quantité au contraire réduite d'AMPc pouvait diminuer l'activité de ce promoteur et ainsi diminuer la sur-expression de PMP22. La sévérité du phénotype semblait par ailleurs dépendre du niveau de sur-expression, avec un effet de seuil. En effet, une sur-expression de 70 % ne semble pas pathogène alors qu'une sur-expression de 100% (patients CMT1A) semble l'être. Dans le cadre de la présente invention, les inventeurs ont testé l'action de la vitamine C sur le pool endogène d'AMPc et ont constaté que la diminution de ce pool permettait de diminuer l'expression de PMP22, de manière à ce que son taux passe au dessous du seuil de pathogénéicité. Afin de tester cette hypothèse des animaux traités pendant 3 mois avec de la vitamine C et des animaux de la même fratrie auxquels avait été administré un placebo ont été sacrifiés. Les nerfs sciatiques ont été prélevés, l'ARN a été extrait et le niveau d'expression testé en PCR temps réel à l'aide d'amorces spécifiques du transcrit humain. Les résultats montrent que le niveau d'expression de PMP22 est diminué après le traitement par la vitamine C. Le nerf sciatique des souris non traitées contient huit fois moins d'ARN messager de PMP22 que celui des souris placebo, l'ARN 18S étant utilisé comme témoin.

La vitamine C corrige ainsi, au moins partiellement, la pathologie locomotrice de souris CMT, probablement en diminuant le niveau d'expression de PMP22. La vitamine C, étant par ailleurs bien connue, sa pharmacodynamique et sa toxicité (faible) ayant été étudiées depuis longtemps, des essais cliniques de phase III impliquant cette molécule peuvent être envisagés de manière à répondre à l'attente d'un traitement qu'expriment de nombreux patients atteints par cette pathologie.

### EXEMPLE 1 :

Un comprimé effervescent sécable contient environ :

| **INGREDIENTS** | **FORMULE UNITAIRE (mg)** | | |
|---|---|---|---|
| Acide ascorbique | 250 | 1000 | 5000 |
| Acide citrique | 300 | 1200 | 6000 |
| Bicarbonate de sodium | 150 | 600 | 3000 |
| Carbonate de sodium anhydre | 50 | 200 | 1000 |
| Citrate de sodium | 1 | 4 | 20 |
| Edulcorant | 5 | 20 | 100 |
| Arôme | 20 | 80 | 400 |
| Agent colorant | 10 | 40 | 200 |
| Eau purifiée | qs | qs | qs |

### EXEMPLE 2 :

Une solution injectable contient environ :

| **INGREDIENTS** | **FORMULE UNITAIRE (mg)** |
|---|---|
| Acide ascorbique | 500 |
| Phosphate monosodique dihydraté | qs pH = 6.0 |
| Acide chlorhydrique | qs pH = 6.0 |
| Eau p préj inj | qs 5 ml |

## Revendications

1. Utilisation de la vitamine C ou un de ses dérivés dans le cadre de la préparation d'une composition destinée à la prévention ou au traitement de la maladie de Charcot-Marie-Tooth.

2. Utilisation selon la revendication 1, **caractérisée en ce que** la maladie de Charcot-Marie-Tooth correspond au type 1 (CMT1) de ladite maladie.

3. Utilisation selon l'une des revendications 1 ou 2, **caractérisée en ce que** la vitamine C est choisie dans le groupe comprenant la vitamine C naturelle, la vitamine C synthétique et un mélange de ces dernières.

4. Utilisation selon l'une des revendications 1 et 2, **caractérisée en ce que** le dérivé de la vitamine C est choisi parmi les esters et les sels de la vitamine C.

5. Utilisation selon la revendication 4, **caractérisée en ce que** le dérivé est choisi parmi l'ascorbyl palmitate, le dipalmitate L-ascorbate et leur mélange ou parmi les dérivés glycosylés, mannosylé, fructosylé, fucosylé, galactosylé, N-acétylglucosaminé, N-acétylmuramique de l'acide ascorbique et leurs mélanges, de préférence l'ascorbyl-2 glucoside, le 2-O-alpha-D glucopyranosyl de l'acide ascorbique ou le 6-O-beta-D galactopyranosyl de l'acide L-ascorbique.

6. Utilisation selon la revendication 4, **caractérisée en ce que** le dérivé est choisi parmi les sels métalliques d'acide ascorbique phosphorylés, notamment les ascorbyl phosphates de métal alcalin, les ascorbyl phosphates de métal alcalino-terreux et les ascorbyl phosphates de métal de transition, de préférence l'ascorbyl phosphate de magnésium, ou encore les ascorbyl sulfates.

7. Utilisation selon l'une quelconque des revendications précédentes, **caractérisée en ce que** la composition comprend de 250 milligrammes à 6 grammes de vitamine C ou d'un de ses dérivés.

## Claims

1. Use of vitamin C or a derivative thereof for the preparation of a composition for the prevention or treatment of Charcot-Marie-Tooth disease.

2. The use according to claim 1, **characterised in that** Charcot-Marie-Tooth disease is type 1 Charcot-Marie-Tooth disease (CMT1).

3. The use according to claim 1 or 2, **characterised in that** the vitamin C is selected in the group comprising natural vitamin C, synthetic vitamin C and a mixture thereof.

4. The use according to claim 1 or 2, **characterised in that** the vitamin C derivative is selected from vitamin C salts and esters.

5. The use according to claim 4, **characterised in that** the vitamin C derivative is selected from ascorbyl palmitate, dipalmitate L-ascorbate, and mixture thereof or selected from glycosylated, mannosylated, fructosylated, fucosylated, galactosylated, N-acetylglucosaminated, N-acetylmuramic derivatives of ascorbic acid and their mixtures, preferably ascorbyl-2 glucoside, 2-O-alpha-D-glucopyranosyl ascorbic acid and 6-O-beta-D-galactopyranosyl L-ascorbic acid.

6. The use according to claim 4, **characterised in that** the vitamin C derivative is selected from the metal salts of phosphorylated ascorbic acid, including the alkaline metal ascorbyl phosphates, the alkaline earth metal ascorbyl phosphates and the transition metal ascorbyl phosphates, preferably magnesium ascorbyl phosphate, or the ascorbyl sulfates.

7. The method according to anyone of the preceding claims, **characterised in that** the composition comprises from 250 mg to 6 grams of vitamin C or a derivative thereof.

## Patentansprüche

1. Verwendung von Vitamin C oder einem seiner Derivate im Rahmen der Zubereitung einer Zusammensetzung für die Prävention oder die Behandlung der Charcot-Marie-Tooth-Krankheit.

2. Verwendung gemäß Anspruch 1, **dadurch gekennzeichnet, dass** die Charcot-Marie-Tooth-Krankheit dem Typ 1 (CMT1) besagter Krankheit entspricht.

3. Verwendung gemäß einem der Ansprüche 1 oder 2, **dadurch gekennzeichnet, dass** das Vitamin C aus der Gruppe, umfassend das natürliche Vitamin C, das synthetische Vitamin C oder ein Gemisch dieser beiden, ausgewählt ist.

4. Verwendung gemäß einem der Ansprüche 1 oder 2, **dadurch gekennzeichnet, dass** das Derivat des Vitamins C aus den Estern und den Salzen des Vitamins C ausgewählt ist.

5. Verwendung gemäß Anspruch 4, **dadurch gekennzeichnet, dass** das Derivat aus dem Ascorbylpalmitat, dem L-Ascorbyldipalmitat und ihrem Gemisch oder aus den glycosylierten, mannosylierten, fructosylierten, fucosylierten, galactosylierten, N-Acetylglucosamin-, N-Acetylmuramin-Derivaten der Ascorbinsäure und ihren Gemischen, vorzugsweise dem Ascorbyl-2-glucosid, der 2-O-alpha-D-Glucopyranosyl-ascorbinsäure oder der 6-O-beta-D-Galactopyranosyl-L-ascorbinsäure, ausgewählt ist.

6. Verwendung gemäß Anspruch 4, **dadurch gekennzeichnet, dass** das Derivat aus den Metallsalzen der phosphorylierten Ascorbinsäure, insbesondere den Alkalimetall-Ascorbylphosphaten, den Erdalkalimetall-Ascorbylphosphaten und den Übergangsmetall-Ascorbylphosphaten, vorzugsweise dem Magnesium-Ascorbylphosphat, oder auch den Ascorbylsulfaten, ausgewählt ist.

7. Verwendung gemäß einem der vorhergehenden Ansprüche, **dadurch gekennzeichnet, dass** die Zusammensetzung 250 Milligramm bis 6 Gramm Vitamin C oder eines seiner Derivate umfasst.
